# FASCICULE DE BREVET EUROPEEN

(11) **EP 1 172 089 B1**
(45) Date de publication et mention de la délivrance du brevet: **26.03.2003**
(21) Numéro de dépôt: 01401616.6
(22) Date de dépôt: 19.06.2001
(51) Int. Cl.: A61K 7/48

(54) **Emulsion eau-dans-huile et ses utilisations notamment dans le domaine cosmétique**
Wasser-in-Öl Emulsion und ihre Verwendung insbesondere in der Kosmetik
Water-in-oil emulsion and its use especially in cosmetics

(30) Priorité: 13.07.2000 FR 0009223
(43) Date de publication de la demande: 16.01.2002
(73) Titulaire: L'OREAL, 75008 Paris (FR)
(72) Inventeur: Aubrun-Sonneville, Odile, 92160 Antony (FR); Simonnet, Jean-Thierry, 75011 Paris (FR)
(74) Mandataire: Rasson, Catherine

(56) Documents cités:
- EP-A- 0 709 084
- WO-A-01/78888
- DE-A- 19 532 419
- US-A- 4 369 123
- US-A- 4 698 065
- US-A- 4 705 682
- US-A- 4 708 753
- US-A- 5 541 341
- US-A- 5 631 389
- US-A- 5 650 158
- US-A- 5 652 266
- US-A- 5 674 511
- US-A- 5 786 468
- CAREY ET AL.: 'Improved high internal phase emulsions using alkenyl succinic anhydride based emulsifiers' PROCEEDINGS OF THE 5TH WORLD SURFACTANTS CONGRESS 29 Mai 2000 - 02 Juin 2000, FORTEZZA, pages 883 - 888

## Description

La présente invention se rapporte à une émulsion eau-dans-huile, comprenant un oligomère ou un polymère dérivé de polyoléfine, et à ses utilisations comme composition cosmétique, dermatologique ou pharmaceutique. Elle peut être destinée en particulier au soin de la peau, des lèvres et des ongles, au démaquillage et/ou au nettoyage de la peau, et/ou au maquillage de la peau et/ou des lèvres. Elle peut être plus particulièrement utilisée pour le traitement des peaux et/ou lèvres sèches et/ou peaux sensibles.

Dans les domaines cosmétique et dermatologique, il est courant d'utiliser des crèmes constituées d'une émulsion eau-dans-huile (E/H) comportant des globules aqueux dispersés dans une phase huileuse. Ces émulsions comportent une phase continue huileuse et permettent donc de former à la surface de la peau un film lipidique qui prévient la perte d'eau transépidermique, protège la peau des agressions extérieures et peut aussi augmenter la rémanence des filtres solaires. Les émulsions eau-dans-huile permettent aussi de protéger et de véhiculer des actifs hydrophiles sensibles à l'oxydation. Ces émulsions sont particulièrement appropriées pour le soin et la réparation des peaux sèches et déshydratées auxquelles elles apportent confort et protection grâce à la barrière lipidique qu'elles forment sur la peau.

Toutefois, malgré leur grande efficacité, les émulsions E/H sont minoritaires parmi les formes galéniques utilisées dans le domaine cosmétique car elles posent deux problèmes majeurs. Tout d'abord, ces émulsions ont l'inconvénient de manquer généralement d'agrément cosmétique, c'est-à-dire qu'elles sont grasses, lourdes, collantes et manquent de fraîcheur du fait de la phase externe huileuse. Elles sont en général difficiles à appliquer sur la peau, pénètrent difficilement et laissent sur la peau un film rémanent brillant et souvent collant.

Par ailleurs, les émulsions E/H présentent des problèmes de stabilité, notamment quand la phase aqueuse est en quantité importante ou quand l'émulsion est fluide (consistance d'un lait plutôt que d'une crème). Les gouttes de phase aqueuse ont alors tendance à s'agréger et à former des amas visibles au microscope. Cette agrégation est dommageable pour la stabilité des émulsions ; elle favorise d'une part le crémage ou la sédimentation des systèmes fluides, et d'autre part la coalescence des gouttes, conduisant à l'apparition de domaines d'eau, c'est-à-dire de gouttes de phase aqueuse de dimension supérieure à 50 microns. Il est souvent nécessaire, pour stabiliser ces émulsions, d'utiliser un fort taux d'émulsionnants et/ou d'introduire une certaine quantité de facteurs de consistance, tels que les cires. Toutefois, ces ingrédients contribuent à accentuer les défauts cosmétiques (effet poisseux et gras) des émulsions E/H et il en résulte l'obtention de compositions souvent compactes et lourdes. Par ailleurs, en présence de ces facteurs de consistance, il est difficile d'obtenir des émulsions fluides car ces facteurs épaississent les émulsions. De plus, si on augmente fortement la teneur en émulsionnant de ces émulsions pour remédier à leur instabilité, les émulsions obtenues peuvent se montrer irritantes vis-à-vis de certains types de peau, notamment les peaux sensibles.

Ainsi, il existe un grand nombre d'émulsions eau-dans-huile stabilisées par différents tensioactifs, en particulier, des dérivés alkylés de polyglycérol, des polyéthylène glycols alkylés, des dérivés alkylés de sorbitane; des sels métalliques d'acides gras, des tensioactifs siliconés. Toutefois, dans la plupart des émulsions stabilisées par des tensioactifs hydrocarbonés, la teneur en phase aqueuse doit rester inférieure à 80% en masse et le taux de tensioactif doit être relativement élevé, pour pouvoir fabriquer une émulsion de stabilité acceptable. Par ailleurs, il est généralement nécessaire d'augmenter la viscosité de la phase huileuse en ajoutant des cires (voir par exemple DE-A-3430256), des gélifiants huileux (voir par exemple EP-A-795321) ou des argiles modifiées (voir par exemple EP-A-331833) pour améliorer leur stabilité. Les produits qui en résultent manquent souvent de fraîcheur et de légèreté.

En outre, les tensioactifs siliconés peuvent améliorer les propriétés cosmétiques des émulsions eau-dans-huile. Cependant, le toucher reste caractéristique des silicones, et des huiles de silicone doivent généralement être les constituants majoritaires de la phase huileuse. Par ailleurs, généralement, il est nécessaire de coupler ces tensioactifs avec au moins un autre tensioactif, le plus souvent hydrocarboné, pour parfaire la stabilité de l'émulsion.

Il subsiste donc le besoin d'une émulsion eau-dans-huile stable ne présentant pas les inconvénients rencontrés avec celles connues jusqu'à présent, en particulier d'une émulsion au toucher léger et frais, ne contenant pas de tensioactif siliconé, pouvant être fluide et/ou avoir une teneur en phase aqueuse élevée et présentant une bonne stabilité, même en l'absence de facteurs de consistance de la phase huileuse, notamment même en l'absence de cires.

L'émulsion selon l'invention permet de pallier les problèmes mentionnés précédemment. En effet, la demanderesse a trouvé de manière surprenante qu'il était possible d'obtenir une composition sous forme d'émulsion eau-dans-huile ayant de bonnes propriétés cosmétiques (toucher léger, frais et riche à la fois) et une bonne stabilité en utilisant comme émulsionnant, un oligomère ou polymère dérivé de polyoléfine, comportant au moins une partie polaire. En particulier, les dérivés de polyoléfine utilisés selon l'invention permettent de fabriquer des émulsions eau-dans-huile très riches en phase aqueuse (plus de 80%) tout en étant stables, et des émulsions eau-dans-huile de faible viscosité et stables.

La présente invention a pour objet une composition apte à être appliquée par voie topique, comprenant, dans un milieu physiologiquement acceptable, une phase aqueuse dispersée dans une phase huileuse, caractérisée en ce qu'elle comprend comme émulsionnant, au moins un oligomère ou un polymère dérivé de polyoléfine, comportant une partie apolaire polyoléfinique comprenant au moins 40 atomes de carbone et au moins une partie polaire, la quantité d'oligomère(s) ou de polymère(s) émulsionnant(s) allant de 0,1 à 10 % en poids de matière active par rapport au poids total de l'émulsion, la quantité de phase aqueuse allant de 40 et 95 % en poids par rapport au poids total de la composition et la quantité d'eau étant d'au moins 30 % en poids par rapport au poids total de la composition.

On entend ici par « milieu physiologiquement acceptable », un milieu compatible avec la peau et/ou les muqueuses (lèvres).

Les oligomères et polymères utilisables dans l'invention sont connus dans d'autres domaines. Ainsi, ils sont décrits comme stabilisants d'émulsions explosives qui sont des émulsions inverses de nitrate d'ammonium fondu ou de solution saturée de nitrate d'ammonium dans une huile hydrocarbonée. Dans ces émulsions, la phase interne contient au plus 15% en poids d'eau (voir par exemple les documents US-A-4,234,435 et US-A-4,708,875).

Par ailleurs, ces composés sont connus comme stabilisants de compositions fertilisantes sous forme d'émulsion eau-dans-huile (voir le document US-A-5,518,517) en vue d'obtenir un relargage contrôlé des substances fertilisantes. La phase interne est constituée d'au moins 70% en poids de fertilisants et d'au plus 30% en poids d'eau.

En outre, le document WO-A-01/78888 décrit l'utilisation de tels polymères pour la préparation de l'émulsion primaire d'une émulsion E/H/E, notamment pour l'encapsulation d'herbicides.

Par ailleurs, le document de J.M. Carey et al;, publié dans "Proceedings of the 5th World Surfactant Congress", volume 2, Fortezza, 29 Mai - 2 Juin 2000, page 883 décrit l'utilisation d'émulsifiants à base d'anhydride alkenyl succinique dans les émulsions inverses à phase interne concentrée.

Dans les émulsions E/H décrites dans ces documents de l'art antérieur, la phase interne aqueuse contient des substances propres au domaine d'application de la composition (tels que explosifs et fertilisants), solubilisées ou dispersées dans un peu d'eau. Ces documents ne décrivent ni ne suggèrent que ces dérivés de polyoléfines puissent permettre l'obtention d'émulsions eau-dans-huile contenant une forte proportion d'eau et des substances cosmétiques, dermatologiques et/ou pharmaceutiques, dispersées ou solubilisées dans cette quantité importante d'eau, pour donner une composition fraîche, apte à être appliquée par voie topique notamment sur la peau, et stable.

L'utilisation d'oligomères ou de polymères dérivés de polyoléfines comme émulsionnants dans la composition de l'invention permet d'obtenir des émulsions eau-dans-huile constituant des compositions stables de faible viscosité, ainsi que des compositions ayant une bonne stabilité bien que très riches en phase aqueuse (supérieure à 80 % en poids) et notamment riche en eau (au moins 30 % en poids d'eau et de préférence au moins 40 % et mieux au moins 60 % en poids d'eau par rapport au poids total de la composition). La stabilisation de ces compositions ne nécessite ni agents structurants de la phase huileuse (cires, polymères ou argiles modifiées), ni association avec un autre tensioactif. Les quantités d'émulsionnants utilisées sont relativement faibles. En outre, ces d'émulsionnants favorisent l'obtention de compositions fraîches et légères.

La présente invention a aussi pour objet l'utilisation de 0,1 à 10 % en poids de matière active par rapport au poids total de l'émulsion, d'au moins un oligomère ou un polymère dérivé de polyoléfine, comportant une partie apolaire polyoléfinique comprenant au moins 40 atomes de carbone et au moins une partie polaire, pour la préparation d'une émulsion E/H comprenant dans un milieu physiologiquement acceptable, au moins 30 % en poids d'eau par rapport au poids total de la composition et de 40 et 95 % en poids de phase aqueuse par rapport au poids total de la composition.

La stabilité des émulsions selon l'invention se traduit par une bonne dispersion de la phase aqueuse dans la phase huileuse et l'absence d'amas de gouttes aqueuses, favorisant l'apparition de domaines d'eau de diamètre important. Le comportement de l'émulsion peut être mis en évidence par un simple test de dilution de l'émulsion par de l'huile, suivi de son observation au microscope, comme c'est décrit ci-dessous dans les exemples. Dans la plupart des cas, les gouttes sont agrégées et forment des amas, ce qui favorise la sédimentation et la formation de domaines d'eau ; l'émulsion est alors considérée comme instable du fait de son hétérogénéité créée par la présence de ces domaines d'eau. Dans le cas contraire, on observe une bonne dispersion des gouttes de phase aqueuse et une bonne stabilité de l'émulsion qui est homogène.

Ainsi, même en l'absence de composés cireux dans la phase huileuse, les émulsions de l'invention peuvent avoir une texture crème ou fluide tout en ayant une bonne stabilité. La viscosité des émulsions peut varier dans une large mesure et aller notamment de 1 à 150 poises (0,1 Pa.s à 15 Pa.s), ces viscosités étant mesurées à environ 25°C à l'aide du viscosimètre "Rhéomat Metler" qui est, de manière générale, équipé d'un mobile 2 pour les gammes de viscosités inférieures à 7 poises, d'un mobile 3 pour les gammes de viscosités de 2 à 40 poises, et d'un mobile 4 pour les gammes de viscosités de 20 poises à 80 poises.

Les oligomères et polymères utilisés comme émulsionnants dans la composition de l'invention sont constitués d'une partie apolaire polyoléfinique et d'au moins une partie polaire. Ils peuvent présenter une structure de type bloc ou peigne.

La partie apolaire polyoléfinique comprend au moins 40 atomes de carbone et de préférence de 60 à 700 atomes de carbone. Il est important que cette partie comporte au moins 40 atomes de carbone pour atteindre le but de l'invention. Sil y a moins de 40 atomes de carbone, on n'obtient pas un système bien stable. Cette partie apolaire peut être choisie parmi les polyoléfines telles que les oligomères, les polymères et/ou les copolymères d'éthylène, d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptadécène et de 1-octadécéne. Ces polyoléfines sont hydrogénées ou non.

Par ailleurs, les oligomères ou polymères dérivés de polyoléfine utilisés dans la composition de l'invention comportent au moins une partie polaire. Cette partie polaire confère aux dérivés de polyoléfines des propriétés amphiphiles. Ainsi, ces oligomères ou polymères abaissent la tension interfaciale (eau / huile, c'est-à-dire entre phase aqueuse et phase huileuse) d'au moins 10 mN/m quand ils sont présents à une concentration de 0,01% en poids par rapport au poids total de la phase huileuse. Par exemple, la polyoléfine à terminaison succinique décrite ci-après et commercialisée sous la dénomination L2724 par la société Lubrizol, à une concentration de 0,01% en poids par rapport au poids total de la phase huileuse, abaisse la tension interfaciale de 15 mN/m à l'interface d'une phase aqueuse constituée d'une solution aqueuse à 1% de MgSO4, et d'une phase huileuse comportant un mélange d'huiles (isohexadécane/polyisobutène hydrogéné/silicone volatile dans un rapport 8/6/4).

La partie polaire des émulsionnants oligomères ou polymères de l'invention peut être anionique, cationique, non ionique, zwitterionique ou amphotère. Elle est par exemple constituée de polyalkylène glycols ou de polyalkylène imines, ou encore d'acides ou de diacides carboxyliques, de leurs anhydrides ou de leurs dérivés, et leurs mélanges. Des émulsionnants oligomères ou polymères à partie polaire acide carboxylique peuvent être par exemple issus de la réaction entre une polyoléfine et au moins un acide ou anhydride carboxylique choisi dans le groupe comprenant l'acide maléique, l'anhydride maléique, l'acide fumarique, l'acide itaconique, l'acide citraconique, l'acide mésaconique, l'acide aconitique. De préférence, la partie polaire est constituée par l'acide ou l'anhydride succinique, leurs dérivés esters ou amides, les sels d'ions alcalins, alcalino-terreux ou organiques correspondants, ou bien encore par du polyoxyéthylène.

Les émulsionnants dérivés de polyoxyéthylène peuvent être par exemple choisis parmi les polymères diblocs polyisoprène-polyoxyéthylène, les polymères poly(éthylène-co-propylène)-polyoxyéthylène et leurs mélanges. Ces polymères sont décrits dans la publication de Allgaier, Poppe, Willner, Richter (Macromolecules, 1997, vol. 30, p. 1582-1586).

Les émulsionnants dérivés d'acide ou d'anhydride succinique peuvent être choisis notamment parmi les dérivés polyoléfines d'acide ou d'anhydride succinique décrits dans les brevets US-A-4,234,435, US-A-4,708,753, US-A-5,129,972, US-A-4,931,110, GB-A-2,156,799 et US-A-4,919,179. La partie polyoléfine peut être constituée par exemple de polyisobutylène, hydrogéné ou non, de poids moléculaire allant de 400 à 5000. Dans le polyisobutylène à terminaison succinique ainsi obtenu, la partie succinique peut être estérifiée, amidifiée ou sous forme de sel, c'est-à-dire qu'elle peut être modifiée par des alcools, des amines, des alcanolamines ou des polyols, ou encore se trouver sous forme de sels de métal alcalin ou alcalino-terreux, d'ammonium ou encore de base organique comme les sels de diéthanolamine et de triéthanolamine. Les polyoléfines à terminaison succinique estérifiée ou amidifiée sont des produits de réaction de (a) une polyoléfine à terminaison succinique, et de (b) une amine ou un alcool, pour former une amide ou un ester. Le terme « amine » utilisé ici comprend tous types d'amines dont les alcanolamines. Il peut s'agir par exemple de mono-amines primaires, secondaires ou tertiaires, ces amines pouvant être aliphatiques, cycloaliphatiques, aromatiques, hétérocycliques, saturées ou insaturées. Par ailleurs, les alcools peuvent être des mono- ou poly-alcools. Les mono-alcools comprennent les alcools aliphatiques primaires, secondaires ou tertiaires, et les phénols. Les poly-alcools peuvent être par exemple choisis parmi les poly-alcools aliphatique, cycloaliphatiques, aromatiques et hétérocycliques. Les polyoléfines à terminaison succinique modifiée (estérifiée ou amidifiée) et leur procédé de préparation sont décrits en particulier dans le document US-A-4,708,753 qui est incorporé ici pour référence.

Comme polyoléfines à terminaison succinique, on peut citer notamment les polyisobutylène à terminaison succinique modifiée, tels que les produits commercialisés sous les dénominations L2724 et L2721 par la société Lubrizol.

Un autre exemple de tensioactif polymérique utilisable dans l'invention est le produit de la réaction de l'anhydride maléique avec le polyisobutylène, tel que le produit commercialisé sous la dénomination Glissopal SA par la société BASF.

La quantité d'oligomère(s) ou de polymère(s) émulsionnant(s) dans la composition de l'invention peut aller par exemple de 0,1 à 10 % en poids de matière active, de préférence de 0,5 à 5 % en poids et mieux de 1 à 3 % en poids par rapport au poids total de la composition. On peut utiliser un ou plusieurs oligomères ou polymères dérivés de polyoléfines. Selon un mode préféré de réalisation de l'invention, les oligomères ou polymères dérivés de polyoléfines sont les seuls émulsionnants utilisés dans la composition selon l'invention. Toutefois, on peut éventuellement, ajouter d'autres agents amphiphiles habituellement utilisés dans les émulsions eau-dans-huile, tels que les tensioactifs classiques ioniques, non ioniques, amphotères, zwitterioniques, les oligomères ou les polymères amphiphiles, les particules organiques ou inorganiques amphiphiles.

La quantité de phase aqueuse dans l'émulsion selon l'invention est de préférence d'au moins 40 % en poids par rapport au poids total de la composition. Elle peut aller par exemple de 40 à 95 % en poids de préférence de 50 à 90 %, mieux de 60 à 90 % en poids et encore mieux de 80 à 90 % en poids par rapport au poids total de la composition. La composition de l'invention contient de préférence au moins 30 % en poids d'eau et mieux au moins 50 % en poids d'eau, par rapport au poids total de la composition. La phase aqueuse comprend de l'eau et tous les adjuvants hydrosolubles ou hydrodispersibles éventuellement présents, tels que par exemple les polyols comme la glycérine et les glycols, les alcools inférieurs en C2 à C6 comme l'éthanol, les polymères tels que les carbomers et les polysaccharides, les sels comme le sulfate de magnésium, le chlorure de magnésium ou de sodium, les sucres comme le glucose et le fructose.

La phase huileuse peut comprendre tous les corps gras classiquement utilisés dans le domaine cosmétique, et notamment les huiles, et les additifs lipophiles tels que les acides gras, les alcools gras et les gommes.

Parmi les huiles utilisables dans l'émulsion de l'invention, on peut citer par exemple les huiles végétales telles que l'huile de noyaux d'abricot, l'huile d'avocat, l'huile de noix de macadamia, l'huile de tournesol, l'huile d'olive et l'huile de soja ; les huiles minérales comme l'huile de vaseline ; les huiles de synthèse comme le polyisobutène hydrogéné, les esters d'acides gras et d'alcool gras (en C6-C30), les éthers d'alcool gras (en C4 à C30 saturés et/ou ramifiés); les huiles de silicone, notamment les huiles de silicone volatiles comme les cyclométhicones (cyclotétradiméthylsiloxane ou cyclotétraméthicone, cyclopentadiméthylsiloxane ou cyclopentaméthicone, cyclohexadiméthylsiloxane ou cyclohexaméthicone); les huiles fluorées et leurs mélanges.

Selon un mode particulier de réalisation de l'invention, la phase huileuse de l'émulsion selon l'invention contient au moins 50% d'une ou plusieurs huiles hydrocarbonées, c'est-à-dire d'huiles ne comprenant que du carbone et de l'hydrogène, qui peuvent être volatiles ou non volatiles et être minérales ou de synthèse. Comme huiles hydrocarbonées, on peut citer par exemple le squalane, le polyisobutène hydrogéné et les huiles hydrocarbonées à chaîne ramifiée qui comportent de préférence de 6 à 20 et mieux de 6 à 18 atomes de carbone et peuvent être choisies par exemple dans le groupe comprenant l'isohexadécane, l'isododécane, les isoparaffines et leurs mélanges.

Quand l'émulsion est utilisée comme produit de démaquillage de la peau et/ou des yeux, elle contient plus particulièrement des huiles démaquillantes et notamment celles choisies parmi les esters d'acide gras comportant au moins 12 atomes de carbone. Ces esters sont de préférence obtenus à partir d'un alcool à chaîne droite ou ramifiée, comportant de 1 à 17 atomes de carbone et d'un acide gras à chaîne droite ou ramifiée, comportant au moins 12 atomes de carbone et de préférence de 14 à 22 atomes de carbone. Il s'agit de préférence de mono- ou di-esters. Les huiles démaquillantes peuvent être notamment choisies dans le groupe comprenant le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges.

La phase huileuse peut également contenir des cires, des gommes silicones, des gélifiants huileux, des particules organiques ou minérales.

La quantité de phase huileuse dans l'émulsion de l'invention peut aller par exemple de 2,5 à 60 %, de préférence de 5 à 50 % et mieux de 7,5 à 40 % en poids par rapport au poids total de la composition. La ou les huiles hydrocarbonées peuvent représenter tout ou une partie de cette phase huileuse, et elles représentent de préférence au moins 40 % en poids par rapport au poids total de la phase huileuse.

La composition de l'invention est une émulsion E/H. Cette émulsion peut être utilisée telle quelle ou être utilisée pour la préparation d'émulsion multiple E/H/E par incorporation de l'émulsion E/H primaire dans une phase aqueuse exteme. Ainsi, les oligomères ou polymères dérivés de polyoléfine décrits ci-dessus peuvent être utilisés aussi comme émulsionnants d'émulsion multiple E/H/E. Par ailleurs, ils peuvent être utilisés aussi comme émulsionnants d'émulsion multiple H/E/H, obtenue par incorporation d'une émulsion H/E dans une phase huileuse contenant un ou plusieurs oligomères ou polymères dérivés de polyoléfine.

La présente invention a aussi pour objet l'utilisation d'au moins un oligomère ou un polymère dérivé de polyoléfine, comportant une partie apolaire polyoléfinique comprenant au moins 40 atomes de carbone et au moins une partie polaire, pour la préparation d'une émulsion multiple E/H/E ou H/E/H.

La composition de l'invention peut constituer notamment une composition cosmétique, dermatologique ou pharmaceutique et plus particulièrement une composition cosmétique destinée à une application sur la peau et/ou les muqueuses.

De façon connue, la composition de l'invention peut contenir également des adjuvants habituels dans le domaine cosmétique et/ou dermatologique, autres que ceux cités précédemment, tels que les solvants, les actifs, les conservateurs, les antioxydants, les agents complexants, les parfums, les charges, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques. Bien entendu l'homme du métier veillera à choisir les éventuels additifs complémentaires et/ou leur quantité de telle manière que les propriétés avantageuses de la composition selon l'invention ne soient pas ou substantiellement pas altérées par l'adjonction envisagée.

Comme actifs, on peut citer notamment, outre ceux indiqués plus haut, les hydratants et par exemple les hydrolysats de protéines et les polyols tels que la glycérine, les glycols comme les polyéthylène glycols, et les dérivés de sucre ; les extraits naturels ; les oligomères procyannidoliques ; les vitamines ; l'urée ; la caféine ; les dépigmentants tels que l'acide kojique et l'acide caféique ; les bêta-hydroxyacides tels que l'acide salicylique et ses dérivés ; les alpha-hydroxyacides tels que l'acide lactique et l'acide glycolique ; les rétinoïdes tels que le rétinol et ses dérivés et les caroténoïdes ; les filtres organiques et inorganiques; l'hydrocortisone ; la DHEA ; la mélatonine ; les extraits d'algues, de champignons, de végétaux, de levures, de bactéries ; les protéines, hydrolysées, partiellement hydrolysées ou non ; les enzymes et leurs mélanges.

Le ou les actifs peuvent être par exemple présents en une concentration allant de 0,01 à 20 %, de préférence de 0,1 à 5 % et mieux de 0,5 à 3 % du poids total de la composition.

De manière avantageuse, l'émulsion de l'invention est réalisée par incorporation de la phase aqueuse dans la phase huileuse comprenant les émulsionnants, sous agitation, à une température allant de préférence d'environ 20 à 60°C.

La composition selon l'invention trouve son application dans un grand nombre de traitements, notamment cosmétiques, de la peau et des lèvres notamment pour le traitement, la protection, le soin, le démaquillage, le nettoyage de la peau et/ou le maquillage de la peau et/ou des lèvres. Elle peut être destinée aussi au traitement des peaux sèches et/ou des lèvres sèches.

La composition selon l'invention peut par exemple être utilisée comme produit de soin, de démaquillage et/ou de nettoyage pour le visage sous forme de crèmes ou de laits ou comme produits de maquillage (peau et lèvres), et par exemple fonds de teint, par incorporation de charges ou de colorants.

Aussi, l'invention a encore pour objet l'utilisation cosmétique de la composition telle que définie ci-dessus pour le traitement, la protection, le soin, le démaquillage, le nettoyage et/ou pour le maquillage de la peau et/ou des lèvres.

L'invention a aussi pour objet un procédé de traitement cosmétique de la peau, et/ou des lèvres, caractérisé par le fait qu'on applique sur la peau et/ou les lèvres, une composition telle que définie ci-dessus.

L'invention a aussi pour objet l'utilisation de la composition telle que définie ci-dessus pour la fabrication d'une composition destinée au soin des peaux sèches et/ou des lèvres sèches et/ou des peaux sensibles.

Les exemples ci-après d'émulsions selon l'invention sont donnés à titre d'illustration et sans caractère limitatif. Les quantités y sont données en % en poids, sauf mention contraire.

### EXEMPLE 1 CRÈME HYDRATANTE

| *Phase huileuse :* | |
|---|---|
| L2724 | 2,5 % |
| Isohexadécane | 3,29 % |
| Polyisobutène hydrogéné | 2,47 % |
| Cyclométhicone | 1,64 % |
| Conservateur | 0,1 % |

| *Phase aqueuse :* | |
|---|---|
| Sulfate de magnésium | 0,9 % |
| Conservateurs | 0,65 % |
| Eau | 88,45 % |

La formule se présente sous la forme d'une crème souple, stable au moins deux mois de 4 à 45°C.

### EXEMPLE COMPARATIF 1 :

| *Phase huileuse :* | |
|---|---|
| Artacel 1690 (société ICI) | 2,5 % |
| Isohexadécane | 3,29 % |
| Polyisobutène hydrogéné | 2,47 % |
| Cyclométhicone | 1,64 % |
| Conservateur | 0,1 % |

| *Phase aqueuse :* | |
|---|---|
| Sulfate de magnésium | 0,9 % |
| Conservateurs | 0,65 % |
| Eau | 88,45 % |

La composition se présente sous la forme d'une crème présentant de nombreux domaines d'eau, ayant une taille supérieure à 50 µm (observation au microscope). Cette composition n'est pas stable dans le temps.

### Comparaison de stabilité entre la composition selon l'invention (exemple 1) et une composition de l'art antérieur (exemple comparatif 1) :

### 1) Observation au microscope

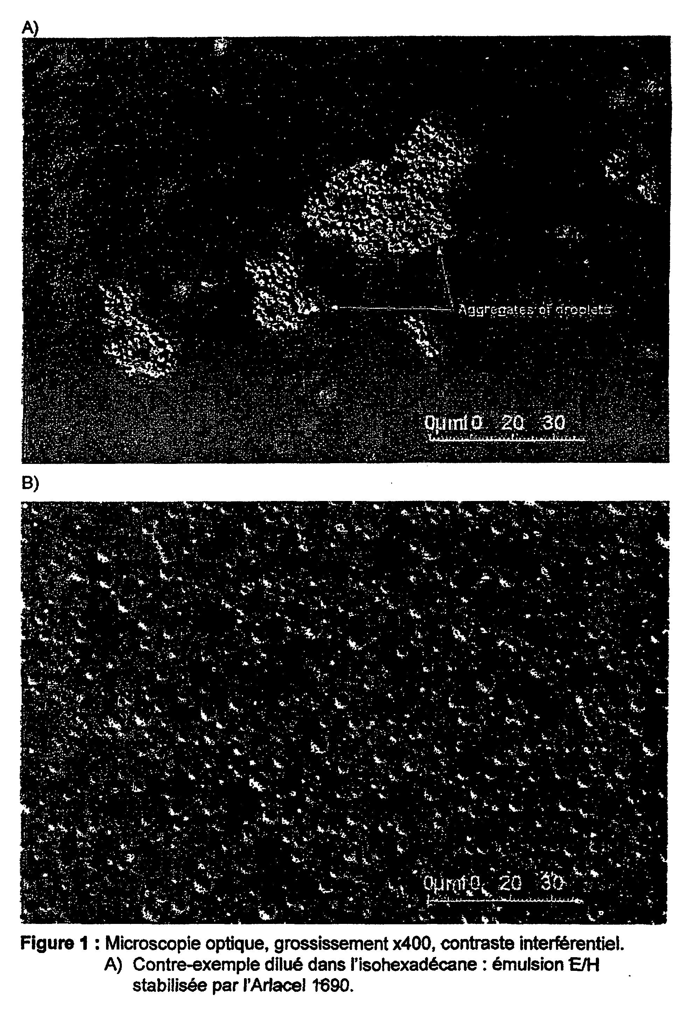

On a préparé une dilution en diluant une goutte d'émulsion avec 5 ml d'isohexadécane. La figure 1 de la présente demande montre les photos pour l'émulsion de l'exemple comparatif (A) et celle de l'exemple 1 selon l'invention (B), observées au microscope après dilution. Pour l'exemple comparatif (A), on observe des amas de globules de phase aqueuse (domaines d'eau) dans la phase huileuse continue, alors que, pour la composition de l'invention (B), on observe une bonne dispersion des globules de la phase aqueuse dans la phase huileuse continue. Ainsi, l'utilisation du mélange d'isostéarate de sorbitane et d'isostéarate de polyglycérol-3 (Arlacel 1690) dans l'exemple comparatif ne permet pas d'obtenir une dispersion stable homogène, alors que l'utilisation d'un émulsionnant polymère (L2724) dans l'exemple de l'invention permet d'obtenir une dispersion stable et homogène.

### 2) Rhéologie

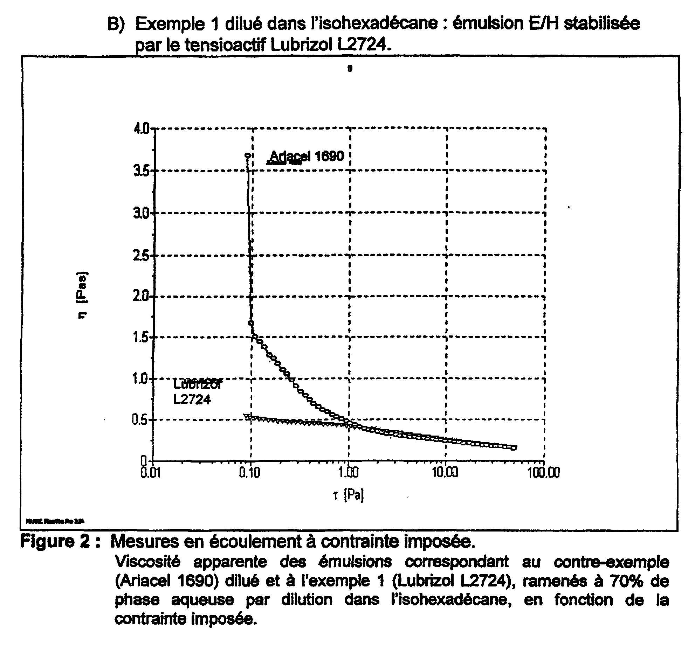

Les résultats de rhéologie permettent également de mettre en évidence la différence entre l'émulsion selon l'invention et celle de l'exemple comparatif, et notamment de montrer l'état agrégé des globules de phase aqueuse dans l'émulsion de cet exemple comparatif. Les systèmes agrégés présentent une viscosité à basse contrainte, supérieure à celle des systèmes non agrégés. Sur la figure 2 jointe à la présente demande, sont présentées les mesures en écoulement à contrainte imposée de l'exemple 1 de l'invention et de l'exemple comparatif 1, après dilution des émulsions avec de l'isohexadécane, dilution telle que la teneur en phase aqueuse soit ramenée à 70% en poids par rapport à la composition totale, afin de mieux mettre en évidence les différences de comportement rhéologique. On observe que le système à l'Arlacel 1690 (exemple comparatif) présente une viscosité à basse contrainte, supérieure à celle du système avec le L2724. Le système à l'Arlacel 1690 est agrégé contrairement au système au L2724.

### EXEMPLE 2 : LAIT HYDRATANT

| *Phase huileuse :* | |
|---|---|
| L2721 | 2,21 % |
| Isohexadécane | 7,87 % |
| Cyclohexaméthicone | 3,93 % |
| Polyisobutène hydrogéné | 5,9 % |
| Conservateurs | 0,09 % |

| *Phase aqueuse :* | |
|---|---|
| Sulfate de magnésium | 0,8 % |
| Conservateurs | 0,58 % |
| Eau | qsp 100 % |

La composition se présente sous forme d'un lait beige, ayant une viscosité de 15,2 poises (1,52 Pa.s) (mobile 3, 200s⁻¹), présentant une bonne stabilité (stable au moins deux mois de 4 à 45°C).

### EXEMPLE 3 CREME DE SOIN POUR PEAUX SECHES

| *Phase huileuse :* | |
|---|---|
| L2724 | 2,24 % |
| Huile de noyaux d'abricot | 10,24 % |
| Isododécane | 6,63 % |
| Conservateur | 0,09 % |

| *Phase aqueuse :* | |
|---|---|
| Sulfate de magnésium | 0,81 % |
| Conservateurs | 0,58 % |
| Eau | qsp 100 % |

La composition se présente sous la forme d'une crème souple, ayant une viscosité de 44 poises (4,4 Pa.s) (mobile 3,200s⁻¹), stable au moins deux mois de 4 à 45°C.

## Revendications

1. Composition apte à être appliquée par voie topique, comprenant, dans un milieu physiologiquement acceptable, une phase aqueuse dispersée dans une phase huileuse, **caractérisée en ce qu'**elle comprend comme émulsionnant, au moins un oligomère ou un polymère dérivé de polyoléfine, comportant une partie apolaire polyoléfinique comprenant au moins 40 atomes de carbone et au moins une partie polaire, la quantité d'oligomère(s) ou de polymère(s) émulsionnant(s) allant de 0,1 à 10 % en poids de matière active par rapport au poids total de l'émulsion, la quantité de phase aqueuse allant de 40 et 95 % en poids par rapport au poids total de la composition et la quantité d'eau étant d'au moins 30 % en poids par rapport au poids total de la composition.

2. Composition selon la revendication 1, **caractérisée en ce que** la partie apolaire polyoléfinique comprend de 60 à 700 atomes de carbone.

3. Composition selon la revendication 1 ou 2, **caractérisée en ce que** la partie apolaire polyoléfinique est choisie parmi les oligomères, les polymères et/ou les copolymères d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptadécène et de 1-octadécéne.

4. Composition selon l'une quelconque des revendications précédentes, **caractérisée en ce que** l'oligomère ou le polymère dérivé de polyoléfine abaisse la tension interfaciale entre phase aqueuse et phase huileuse, d'au moins 10 mN/m quand ledit oligomère ou polymère est présent à une concentration de 0,01% en poids par rapport au poids de phase huileuse.

5. Composition selon l'une quelconque des revendications précédentes, **caractérisée en ce que** la partie polaire est anionique, cationique, non ionique, zwitterionique ou amphotère.

6. Composition selon l'une quelconque des revendications précédentes, **caractérisée en ce que** la partie polaire est choisie dans le groupe comprenant les polyalkylène glycols, les polyalkylène imines, les acides ou diacides carboxyliques, leurs anhydrides ou dérivés, et leurs mélanges.

7. Composition selon l'une quelconque des revendications précédentes, **caractérisée en ce que** la partie polaire est choisie dans le groupe comprenant le polyoxyéthylène, l'acide ou l'anhydride succinique et leurs dérivés.

8. Composition selon l'une quelconque des revendications précédentes, **caractérisée en ce que** l'oligomère ou le polymère dérivé de polyoléfine est issu de la réaction entre un dérivé de polyoléfine et au moins un acide choisi dans le groupe comprenant l'acide maléique ; l'anhydride maléique ; l'acide fumarique ; l'acide itaconique ; l'acide citraconique ; l'acide mésaconique ; l'acide aconitique ; leurs dérivés et leurs mélanges.

9. Composition selon l'une quelconque des revendications précédentes, **caractérisée en ce que** l'émulsionnant est un polyisobutylène à terminaison succinique éventuellement modifiée.

10. Composition selon l'une quelconque des revendications précédentes, **caractérisée en ce que** l'émulsionnant est le produit de la réaction de l'anhydride maléique avec le polyisobutylène.

11. Composition selon l'une quelconque des revendications précédentes, **caractérisée en ce que** la phase huileuse comprend au moins une huile hydrocarbonée.

12. Composition selon la revendication précédente, **caractérisée en ce que** la quantité de phase huileuse va de 2,5 à 60 % en poids par rapport au poids total de la composition.

13. Emulsion selon l'une quelconque des revendications précédentes, **caractérisée par le fait qu'**elle constitue une composition cosmétique.

14. Utilisation cosmétique de la composition selon l'une quelconque des revendications précédentes pour le traitement, la protection, le soin, le démaquillage, le nettoyage et/ou pour le maquillage de la peau et/ou des lèvres.

15. Procédé de traitement cosmétique de la peau et/ou des lèvres, **caractérisé par le fait qu'**on applique sur la peau et/ou les lèvres, une composition selon l'une quelconque des revendications 1 à 12.

16. Utilisation de la composition selon l'une quelconque des revendications 1 à 12 pour la fabrication d'une composition destinée au soin des peaux sèches et/ou des lèvres sèches et/ou des peaux sensibles.

17. Utilisation de 0,1 à 10 % en poids de matière active par rapport au poids total de l'émulsion, d'au moins un oligomère ou un polymère dérivé de polyoléfine, comportant une partie apolaire polyoléfinique comprenant au moins 40 atomes de carbone et au moins une partie polaire, pour la préparation d'une émulsion E/H comprenant dans un milieu physiologiquement acceptable, au moins 30 % en poids d'eau par rapport au poids total de la composition et de 40 et 95 % en poids de phase aqueuse par rapport au poids total de la composition.

18. Utilisation d'au moins un oligomère ou un polymère dérivé de polyoléfine, comportant une partie apolaire polyoléfinique comprenant au moins 40 atomes de carbone et au moins une partie polaire, pour la préparation d'une émulsion multiple E/H/E ou H/E/H constituant une composition cosmétique.

## Claims

1. Composition, capable of being applied topically, comprising, in a physiologically acceptable medium, an aqueous phase dispersed in an oily phase, **characterized in that** it comprises, as emulsifier, at least one oligomer or one polymer derived from a polyolefin, comprising a polyolefinic apolar part comprising at least 40 carbon atoms and at least a polar part, the quantity of emulsifying oligomer(s) or polymer(s) ranging from 0.1 to 10% by weight of active substance relative to the total weight of the emulsion, the quantity of aqueous phase ranging from 40 to 95% by weight relative to the total weight of the composition and the quantity of water being at least 30% by weight relative to the total weight of the composition.

2. Composition according to Claim 1, **characterized in that** the polyolefinic apolar part comprises from 60 to 700 carbon atoms.

3. Composition according to Claim 1 or 2,
**characterized in that** the polyolefinic apolar part is chosen from oligomers, polymers and/or copolymers of ethylene, propylene, 1-butene, isobutene, 1-pentene, 2-methyl-1-butene, 3-methyl-1-butene, 1-hexene, 1-heptene, 1-octene, 1-decene, 1-undecene, 1-dodecene, 1-tridecene, 1-tetradecene, 1-pentadecene, 1-hexadecene, 1-heptadecene and 1-octadecene.

4. Composition according to any one of the preceding claims, **characterized in that** the oligomer or polymer derived from a polyolefin reduces the interfacial tension between the aqueous phase and the oily phase by at least 10 mN/m when the said oligomer or polymer is present at a concentration of 0.01% by weight relative to the weight of the oily phase.

5. Composition according to any one of the preceding claims, **characterized in that** the polar part is anionic, cationic, nonionic, zwitterionic or amphoteric.

6. Composition according to any one of the preceding claims, **characterized in that** the polar part is chosen from the group consisting of polyalkylene glycols, polyalkyleneimines, carboxylic or dicarboxylic acids, anhydrides or derivatives thereof, and mixtures thereof.

7. Composition according to any one of the preceding claims, **characterized in that** the polar part is chosen from the group consisting of polyoxyethylene, succinic acid or anhydride and derivatives thereof.

8. Composition according to any one of the preceding claims, **characterized in that** the oligomer or polymer derived from a polyolefin is obtained from the reaction between a polyolefin derivative and at least one acid chosen from the group consisting of maleic acid; maleic anhydride; fumaric acid; itaconic acid; citraconic acid; mesaconic acid; aconitic acid; derivatives thereof and mixtures thereof.

9. Composition according to any one of the preceding claims, **characterized in that** the emulsifier is a polyisobutylene with an optionally modified succinic ending.

10. Composition according to any one of the preceding claims, **characterized in that** the emulsifier is the product of the reaction of maleic anhydride with polyisobutylene.

11. Composition according to any one of the preceding claims, **characterized in that** the oily phase comprises at least one hydrocarbon oil.

12. Composition according to the preceding claim, **characterized in that** the quantity of oily phase ranges from 2.5 to 60% by weight relative to the total weight of the composition.

13. Emulsion according to any one of the preceding claims, **characterized in that** it constitutes a cosmetic composition.

14. Cosmetic use of the composition according to any one of the preceding claims for the treatment of, protection of, care of, removal of make-up from, cleansing of and/or application of make-up to the skin and/or the lips.

15. Method for the cosmetic treatment of the skin and/or the lips, **characterized in that** a composition according to any one of Claims 1 to 12 is applied to the skin and/or the lips.

16. Use of the composition according to any one of Claims 1 to 12, for the manufacture of a composition intended for the care of dry skin and/or dry lips and/or sensitive skin.

17. Use of 0.1 to 10% by weight of active substance relative to the total weight of the emulsion, at least one oligomer or one polymer derived from a polyolefin, comprising a polyolefinic apolar part comprising at least 40 carbon atoms and at least one polar part, for the preparation of a W/O emulsion comprising, in a physiologically acceptable medium, at least 30% by weight of water relative to the total weight of the composition and from 40 to 95% by weight of aqueous phase relative to the total weight of the composition.

18. Use of at least one oligomer or one polymer derived from a polyolefin, comprising a polyolefinic apolar part comprising at least 40 carbon atoms and at least one polar part, for the preparation of a multiple W/O/W or O/W/O emulsion which constitutes a cosmetic composition.

## Patentansprüche

1. Zusammensetzung, die zur Anwendung auf topischem Wege geeignet ist und die in einem physiologisch akzeptablen Medium eine in einer Ölphase dispergierte wässerige Phase enthält, **dadurch gekennzeichnet, dass** sie als Emulgator mindestens ein von einem Polyolefin abgeleitetes Oligomer oder Polymer enthält, das eine apolare polyolefinische Gruppe mit mindestens 40 Kohlenstoffatomen und mindestens eine polare Gruppe enthält, wobei der Mengenanteil des (der) emulgierenden Oligomers (Oligomere) oder des (der) emulgierenden Polymers (Polymere) ausgedrückt als Wirkstoff im Bereich von 0,1 bis 10 Gew.-%, bezogen auf das Gesamtgewicht der Emulsion, liegt, der Mengenanteil der wässerigen Phase im Bereich von 40 bis 95 Gew.-%, bezogen auf das Gesamtgewicht der Zusammensetzung, liegt und der Wassergehalt mindestens 30 Gew.-%, bezogen auf das Gesamtgewicht der Zusammensetzung, beträgt.

2. Zusammensetzung nach Anspruch 1, **dadurch gekennzeichnet, dass** die apolare polyolefinische Gruppe 60 bis 700 Kohlenstoffatome aufweist.

3. Zusammensetzung nach Anspruch 1 oder 2, **dadurch gekennzeichnet, dass** die apolare polyolefinische Gruppe unter den Oligomeren, Polymeren und/oder Copolymeren von Ethylen, Propylen, 1-Buten, Isobuten, 1-Penten, 2-Methyl-1-buten, 3-Methyl-1-buten, 1-Hexen, 1-Hepten, 1-Octen, 1-Decen, 1-Undecen, 1-Dodecen, 1-Tridecen, 1-Tetradecen, 1-Pentadecen, 1-Hexadecen, l'Heptadecen und 1-Octadecen ausgewählt ist.

4. Zusammensetzung nach einem der vorhergehenden Ansprüche, **dadurch gekennzeichnet, dass** das von einem Polyolefin abgeleitete Oligomer oder Polymer die Grenzflächenspannung zwischen der wässerigen Phase und der Ölphase um mindestens 10 mN/m herabsetzt, wenn das Oligomer oder Polymer in einer Konzentration von 0,01 Gew.-%, bezogen auf das Gewicht der Ölphase, vorliegt.

5. Zusammensetzung nach einem der vorhergehenden Ansprüche, **dadurch gekennzeichnet, dass** die polare Gruppe anionisch, kationisch, nichtionisch, zwitterionisch oder amphoter ist.

6. Zusammensetzung nach einem der vorhergehenden Ansprüche, **dadurch gekennzeichnet, dass** die polare Gruppe unter Polyalkylenglykolen, Polyalkyleniminen, Carbonsäuren oder Dicarbonsäuren, den Anhydriden oder Derivaten dieser Säuren und den Gemischen dieser Verbindungen ausgewählt ist.

7. Zusammensetzung nach einem der vorhergehenden Ansprüche, **dadurch gekennzeichnet, dass** die polare Gruppe unter Polyoxyethylen, Bernsteinsäure oder Bernsteinsäureanhydrid und deren Derivaten ausgewählt ist.

8. Zusammensetzung nach einem der vorhergehenden Ansprüche, **dadurch gekennzeichnet, dass** das von einem Polyolefin abgeleitete Oligomer oder Polymer aus der Umsetzung eines Polyolefinderivats mit mindestens einer Säure stammt, die unter Maleinsäure, Maleinsäureanhydrid, Fumarsäure, Itaconsäure, Citraconsäure, Mesaconsäure, Aconitsäure, den Derivaten dieser Säuren und den Gemischen dieser Verbindungen ausgewählt ist.

9. Zusammensetzung nach einem der vorhergehenden Ansprüche, **dadurch gekennzeichnet, dass** der Emulgator ein Polyisobutylen mit einer gegebenenfalls modifizierten endständigen Bernsteinsäuregruppe ist.

10. Zusammensetzung nach einem der vorhergehenden Ansprüche, **dadurch gekennzeichnet, dass** der Emulgator das Produkt der Umsetzung von Maleinsäureanhydrid mit Polyisobutylen ist.

11. Zusammensetzung nach einem der vorhergehenden Ansprüche, **dadurch gekennzeichnet, dass** die Ölphase mindestens ein Kohlenwasserstofföl enthält.

12. Zusammensetzung nach dem vorhergehenden Anspruch, **dadurch gekennzeichnet, dass** der Mengenanteil der Ölphase im Bereich von 2,5 bis 60 Gew.-%, bezogen auf das Gesamtgewicht der Zusammensetzung, liegt.

13. Emulsion nach einem der vorhergehenden Ansprüche, **dadurch gekennzeichnet, dass** es sich um eine kosmetische Zusammensetzung handelt.

14. Kosmetische Verwendung der Zusammensetzung nach einem der vorhergehenden Ansprüche zur Behandlung, zum Schutz, zur Pflege, zum Abschminken, zur Reinigung und/oder zum Schminken der Haut und/oder der Lippen.

15. Verfahren zur kosmetischen Behandlung der Haut und/oder der Lippen, **dadurch gekennzeichnet, dass** eine Zusammensetzung nach einem der Ansprüche 1 bis 12 auf die Haut und/oder die Lippen aufgebracht wird.

16. Verwendung der Zusammensetzung nach einem der Ansprüche 1 bis 12 zur Herstellung einer Zusammensetzung, die zur Pflege von trockener Haut und/oder trockenen Lippen und/oder empfindlicher Haut bestimmt ist.

17. Verwendung von 0,1 bis 10 Gew.-% (Wirkstoff), bezogen auf das Gesamtgewicht der Emulsion, mindestens eines von einem Polyolefin abgeleiteten Oligomers oder Polymers, das eine apolare polyolefinische Gruppe mit mindestens 40 Kohlenstoffatomen und mindestens eine polare Gruppe aufweist, zur Herstellung eine W/O-Emulsion, die in einem physiologisch akzeptablen Medium mindestens 30 Gew.-% Wasser, bezogen auf das Gesamtgewicht der Zusammensetzung, und 40 bis 95 Gew.-% wässerige Phase, bezogen auf das Gesamtgewicht der Zusammensetzung, enthält.

18. Verwendung mindestens eines von einem Polyolefin abgeleiteten Oligomers oder Polymers, das eine apolare polyolefinische Gruppe mit mindestens 40 Kohlenstoffatomen und mindestens eine polare Gruppe aufweist, zur Herstellung einer multiplen W/O/W-Emulsion oder O/W/O-Emulsion, die eine kosmetische Zusammensetzung bildet.
